# EUROPEAN PATENT APPLICATION

(11) **EP 4 792 857 A1**
(43) Date of publication of application: **19.08.2026**
(21) Application number: 24877331.9
(22) Date of filing: 06.03.2024
(51) Int. Cl.: A61M 5/30, A61M 5/315, A61M 39/24, A61M 5/31

(54) **HANDPIECE HEAD AND LIQUID JET INJECTION DEVICE COMPRISING SAME**

(30) Priority: 12.10.2023 KR 20230136198
(71) Applicant: Kaosys Corp., Cheonan-si, Chungcheongnam-do 31035 (KR)
(72) Inventor: LEE, Soo Yeun, Pohang-si Gyeongsangbuk-do 37800 (KR); BANG, Jin Su, Pohang-si Gyeongsangbuk-do 37800 (KR)
(74) Representative: AWA Sweden AB
(86) International application number: PCT/KR2024/002867
(87) International publication number: WO 2025/079789

(57) **Abstract**

The present disclosure discloses a head for a handpiece configured to discharge a liquid medicine stored therein to the outside when pressurized by an external force. The head for a handpiece includes: an inlet chamber part coupled to a handpiece body and having a liquid medicine filling space therein; a feeder part connected to the inlet chamber part and configured to introduce a liquid medicine into the liquid medicine filling space; a piston mounted inside the inlet chamber part and configured to push the liquid medicine filled in the liquid medicine filling space when pressurized by an external force; an elastic support member configured to elastically support the piston; and an outlet chamber part coupled to the inlet chamber part and configured to discharge the liquid medicine by pressurization of the piston, wherein an end portion of the piston is configured to define a portion of the liquid medicine filling space.

## Description

### Technical Field

The present disclosure relates to a head for a handpiece configured to discharge a liquid medicine stored therein to the outside when pressurized by an external force, and to a liquid jet injection device including the same.

### Background Art

Generally, a technique of accelerating a liquid to cut or penetrate an object has been widely used. A representative example is an ultra-high-pressure cutting method referred to as waterjet cutting. A waterjet is equipment that cuts an object into a desired shape by spraying water or an abrasive mixture compressed at ultra-high pressure onto a surface of the object through an orifice and a nozzle.

Meanwhile, in general, in order to inject a liquid medicine into tissue, a syringe is used. Looking at the structure of the syringe, it is composed of a needle portion inserted into the tissue, a cylinder filled with the liquid medicine, and a piston inserted into and movable within the cylinder. Such a syringe is configured to move the liquid medicine filled in the cylinder by the piston so as to penetrate into the tissue.

Although the syringe is an efficient means for delivering a liquid medicine, issues such as a possibility of infection due to the needle, fear of the needle, and inconvenience of use have been raised, and accordingly, in recent years, research on a needle-free injector has been continuously conducted.

As one example of a needle-free injector, it is possible to configure an energy source capable of pressurizing the piston of the injector cylinder, and by using an orifice and nozzle structure as in a waterjet, to implement a liquid medicine delivery device capable of stably delivering the liquid medicine while continuously supplying the liquid medicine instead of an abrasive mixture. At this time, a high-pressure waterjet stream is mixed with a mixture supplied from the outside and passes through the nozzle at a very high speed. By using this, a structure is possible in which the waterjet is controlled to operate only at a certain moment and the liquid medicine is sequentially supplied from the outside in accordance with the operation sequence.

Meanwhile, among needle-free injectors, there are also methods in which air is compressed using strong air pressure and then pressure is applied to the piston of the cylinder to inject the liquid medicine filled in the cylinder into the skin. In addition, in such conventional methods, an elastic member forming a membrane between the liquid medicine filled in the cylinder and the piston is provided. That is, in the conventional needle-free injector, kinetic energy of the piston pressurized by compressed air is transmitted to the elastic member rather than to the liquid medicine, and the liquid medicine is injected in a manner in which the elastic member is substantially deformed to pressurize the liquid medicine.

According to the structure of such a conventional needle-free injector, the kinetic energy of the piston is transmitted to the liquid medicine through the elastic member, and as a result, a phenomenon may occur in which the accuracy of the energy transmission amount through the piston decreases due to changes in the elastic force of the elastic member over time that vary depending on its manufacturing date, even when the usage period of the injector has elapsed or even when the injector has not been used at all. Consequently, in the conventional needle-free injector, reliability of the accuracy of the liquid medicine administration amount may be reduced.

### Disclosure

### Technical Problem

An object of the present disclosure is to provide a head for a handpiece and a liquid jet injection device including the same, configured such that a magnitude of an external force transmitted to a liquid medicine for discharge of the liquid medicine can be maintained more stably.

### Technical Solution

To achieve the object of the present disclosure, a head for a handpiece according to one embodiment of the present disclosure comprises: an inlet chamber part coupled to a handpiece body and having a liquid medicine filling space therein; a feeder part connected to the inlet chamber part and configured to introduce a liquid medicine into the liquid medicine filling space; a piston mounted inside the inlet chamber part and configured to push a liquid medicine filled in the liquid medicine filling space when pressurized by an external force; an elastic support member configured to elastically support the piston; and an outlet chamber part coupled to the inlet chamber part and configured to discharge the liquid medicine by pressurization of the piston, wherein an end portion of the piston defines a portion of the liquid medicine filling space.

According to one example related to the present disclosure, the end portion of the piston may be formed to be in contact with the liquid medicine filled in the liquid medicine filling space.

According to one example related to the present disclosure, the outlet chamber part may comprise: an outlet chamber body coupled to an inlet chamber body of the inlet chamber part and having a liquid medicine discharge valve mounting groove and a liquid medicine discharge port communicating with the liquid medicine discharge valve mounting groove; and a liquid medicine discharge valve mounted in the liquid medicine discharge valve mounting groove, wherein the liquid medicine discharge valve may be formed to be in contact with the liquid medicine filled in the liquid medicine filling space.

According to one example related to the present disclosure, the liquid medicine discharge valve may be disposed to face the end portion of the piston with the liquid medicine filled in the liquid medicine filling space interposed therebetween.

According to one example related to the present disclosure, the inlet chamber part may comprise: an inlet chamber body detachably formed on the handpiece body and having a piston mounting groove and a liquid medicine injection valve mounting groove respectively communicating with the liquid medicine filling space at different positions; and a liquid medicine injection valve mounted in the liquid medicine injection valve mounting groove, wherein the feeder part may be disposed corresponding to the liquid medicine injection valve.

According to one example related to the present disclosure, the piston may comprise: a piston head exposed to an outside of the inlet chamber part and configured to receive the external force; a piston rod extending from the piston head, having an end portion defining the liquid medicine filling space, and having at least one O-ring groove; and an O-ring mounted in the O-ring groove.

According to one example related to the present disclosure, the elastic support member may be formed to surround the piston rod and may be supported by the piston head and the piston mounting groove facing each other, respectively.

According to one example related to the present disclosure, the elastic support member may have a ring shape having a hollow portion in which the piston rod is accommodated.

A liquid jet injection device according to another embodiment of the present disclosure comprises: a handpiece body; and the head for a handpiece coupled to the handpiece body, wherein the handpiece body comprises: a cylinder unit including a cylinder, a casing surrounding the cylinder, and a hammer accommodated inside the cylinder and configured to move between one end and the other end of the cylinder; a control valve unit configured to selectively open and close an intake passage connecting a compressed air generation unit generating compressed air supplied into the cylinder and one end of the cylinder, and an exhaust passage connecting one end of the cylinder and an outside; and a pressing unit including a pressing member facing an opening formed at the other end of the cylinder and coupled to the inlet chamber part, wherein, when the hammer collides with the pressing member, the pressing member is configured to press the piston.

According to one example related to the present disclosure, a charging part may be provided between the casing and the cylinder, may communicate with the other end of the cylinder, and may be configured such that compressed air is compressed and charged therein when the intake passage is opened, wherein the hammer may be configured such that, after moving from one end to the other end of the cylinder by opening of the intake passage, when the exhaust passage is opened, the hammer moves from the other end to the one end of the cylinder by the compressed air compressed and charged in the charging part.

### Advantageous Effects

Effects of the present disclosure obtained through the above-described solution are as follows.

The head for a handpiece comprises: an inlet chamber part having a liquid medicine filling space; a feeder part configured to introduce a liquid medicine into the liquid medicine filling space of the inlet chamber part; a piston mounted in the inlet chamber part and configured to push a liquid medicine filled in the liquid medicine filling space when pressurized by an external force; an elastic support member disposed in a movement path of the piston and configured to elastically support the piston; and an outlet chamber part coupled to the inlet chamber part and configured to discharge the liquid medicine by pressurization of the piston. Here, an end portion of the piston is formed to define a portion of the liquid medicine filling space.

According to such a configuration of the head for a handpiece, the piston that transmits an external force for discharge of the liquid medicine to the liquid medicine filled in the liquid medicine filling space is configured to directly pressurize the liquid medicine filled in the liquid medicine filling space. That is, the head for a handpiece of the present disclosure does not include a separate configuration for transmitting the external force of the piston to the liquid medicine filling space between the end portion of the piston and the liquid medicine filling space. Accordingly, the external force applied to the piston for discharge of the liquid medicine may be transmitted directly to the liquid medicine filling space.

As a result, by forming the magnitude of the external force applied to the liquid medicine filling space by the piston to be constant, it is possible to provide a head for a handpiece and a liquid jet injection device including the same having high reliability in terms of accuracy of a liquid medicine administration amount, as compared to a head for a handpiece further including a separate configuration between the piston and the liquid medicine filling space. In addition, it is possible to provide a head for a handpiece and a liquid jet injection device including the same having high energy transmission efficiency by reducing loss of energy transmitted to the liquid medicine by the piston.

### Description of Drawings

FIG. 1 is a perspective view illustrating a state in which a head for a handpiece according to one embodiment of the present disclosure is coupled to a handpiece body.
FIG. 2 is a conceptual view of the head for a handpiece illustrated in FIG. 1 and a liquid jet injection device including the same.
FIG. 3 is a perspective view illustrating an exploded state of the head for a handpiece illustrated in FIG. 1.
FIG. 4 is a perspective view illustrating opening and closing states of a liquid medicine injection valve and a liquid medicine discharge valve illustrated in FIG. 3.
FIG. 5 is a conceptual view illustrating a state in which a portion of the liquid jet injection device illustrated in FIG. 2 is separated.
FIG. 6 is a cross-sectional view taken along line A-A illustrated in FIG. 5.
FIG. 7 is a conceptual view illustrating a state before an intake passage of the liquid jet injection device illustrated in FIG. 2 is opened.
FIG. 8 is a conceptual view illustrating a state in which the intake passage is opened and a hammer moves from one end of a cylinder toward the other end in the liquid jet injection device illustrated in FIG. 7.
FIG. 9 is a conceptual view illustrating a state in which the hammer moves to the other end of the cylinder and collides with a pressing member, and the pressing member moves a predetermined distance due to an impact in the liquid jet injection device illustrated in FIG. 8.
FIG. 10 is a conceptual view illustrating a state in which, after the hammer collides with the pressing member, an exhaust passage is opened and the hammer moves toward one end of the cylinder in the liquid jet injection device illustrated in FIG. 9.
FIG. 11 is a conceptual view illustrating a state in which the hammer moves to one end of the cylinder and returns to an initial position in the liquid jet injection device illustrated in FIG. 10.

### Detailed Description of Embodiments

Hereinafter, a head for a handpiece and a liquid jet injection device including the same according to the present disclosure will be described in more detail with reference to the drawings.

In the present specification, even in different embodiments, identical or similar reference numerals are assigned to identical or similar components, and repeated descriptions thereof will be omitted.

A singular expression includes a plural expression unless the context clearly indicates otherwise.

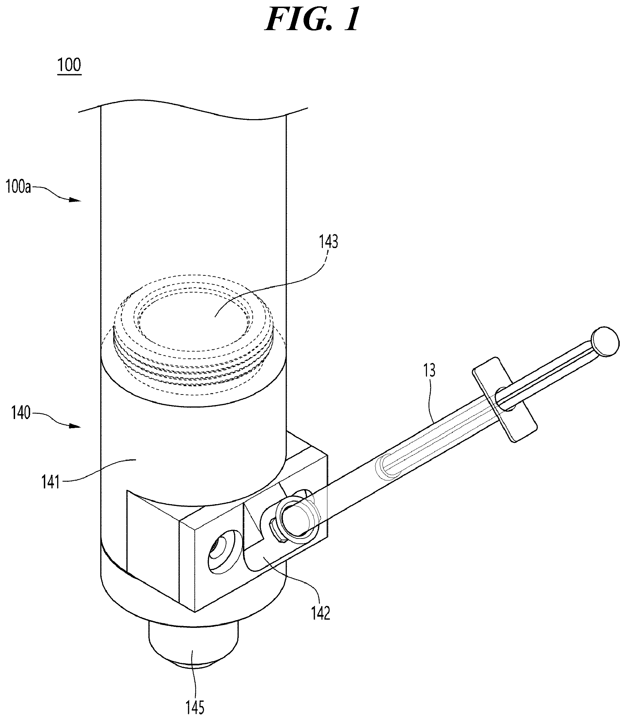

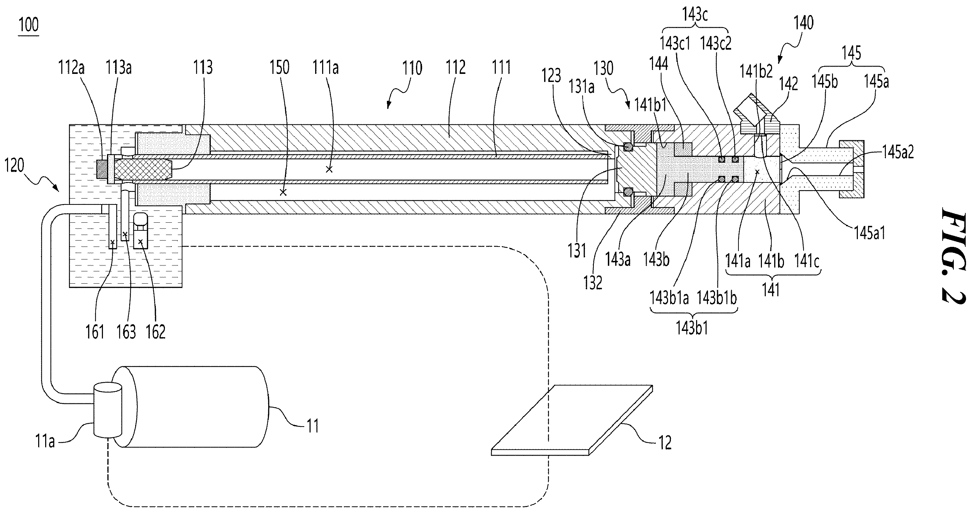

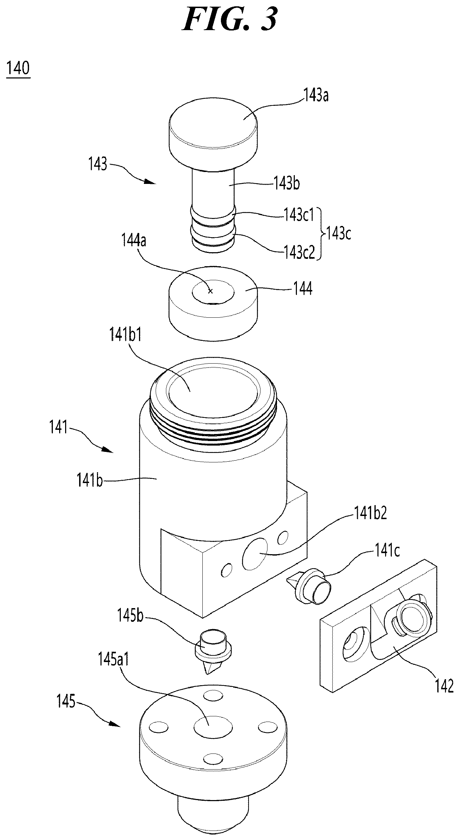

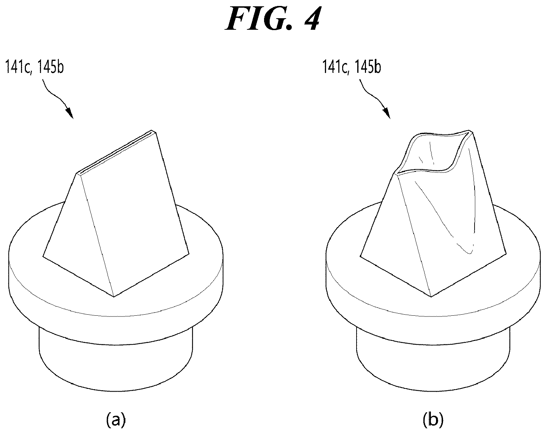

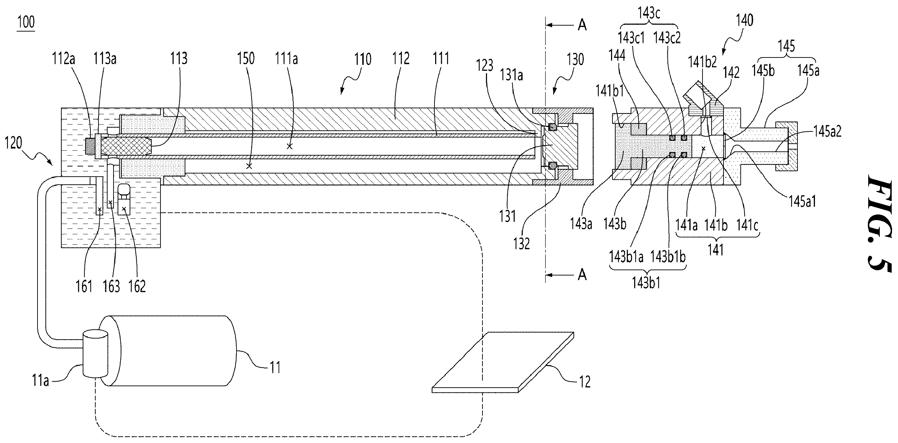

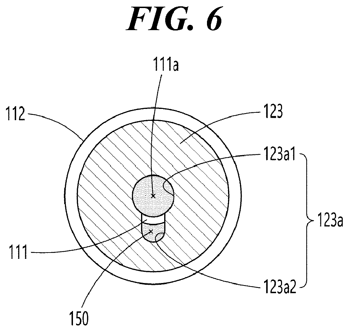

FIG. 1 is a perspective view illustrating a state in which a head for a handpiece (140) according to one embodiment of the present disclosure is coupled to a handpiece body (100a). FIG. 2 is a conceptual view of the head for a handpiece (140) illustrated in FIG. 1 and a liquid jet injection device (100) including the same. FIG. 3 is a perspective view illustrating an exploded state of the head for a handpiece (140) illustrated in FIG. 1. FIG. 4 is a perspective view illustrating opening and closing states of a liquid medicine discharge valve (145b) and a liquid medicine injection valve (141c) illustrated in FIG. 3. FIG. 5 is a conceptual view illustrating a state in which a portion of the liquid jet injection device (100) illustrated in FIG. 2 is separated. FIG. 6 is a cross-sectional view taken along line A-A illustrated in FIG. 5. In addition, FIG. 4(a) illustrates a state in which the liquid medicine discharge valve (145b) and the liquid medicine injection valve (141c) are closed, and FIG. 4(b) illustrates a state in which the liquid medicine discharge valve (145b) and the liquid medicine injection valve (141c) are opened.

Referring to FIGS. 1 to 6, the liquid jet injection device (100) includes the handpiece body (100a) and the head for a handpiece (140).

The handpiece body (100a) is configured to generate an external force for discharge of the liquid medicine and to transmit the external force to the head for a handpiece (140) in which filling and discharge of the liquid medicine are performed. A more detailed description of the handpiece body (100a) will be provided later.

The head for a handpiece (140) includes an inlet chamber part (141), a feeder part (142), a piston (143), an elastic support member (144), and an outlet chamber part (145).

The inlet chamber part (141) is coupled to the handpiece body (100a) and has a liquid medicine filling space (141a) therein. The inlet chamber part (141) may include an inlet chamber body (141b) and a liquid medicine injection valve (141c).

The inlet chamber body (141b) is detachably formed on the handpiece body (100a) and may include a piston mounting groove (141b1) and a liquid medicine injection valve mounting groove (141b2) respectively communicating with the liquid medicine filling space (141a) at different positions. The inlet chamber body (141b) is formed to accommodate the piston (143) therein. The piston (143) may be formed to be movable forward and backward in a state in which the piston is surrounded by the inlet chamber body (141b).

Here, the liquid medicine injection valve (141c) may be configured to be mounted in the liquid medicine injection valve mounting groove (141b2). In addition, at this time, the feeder part (142) may be disposed corresponding to the liquid medicine injection valve (141c). The liquid medicine injection valve (141c) may be formed to selectively open and close a liquid medicine inlet of the inlet chamber body (141b) by a pressure difference.

The feeder part (142) is connected to the inlet chamber part (141) and may be formed to introduce the liquid medicine into the liquid medicine filling space (141a). In addition, as illustrated in FIG. 1, the feeder part (142) may be formed to accommodate the liquid medicine therein, and a syringe configured to supply the accommodated liquid medicine to the feeder part (142) may be connected thereto.

The piston (143) is mounted inside the inlet chamber part (141) and is configured to push the liquid medicine filled in the liquid medicine filling space (141a) when pressurized by an external force. At this time, an end portion of the piston (143) may be configured to directly contact the liquid medicine filled in the liquid medicine filling space (141a) of the inlet chamber part (141). In addition, the external force applied to the piston (143) may be generated by a high-pressure hammer air method, a laser method, or a magnetic method.

In addition, the piston (143) may include a piston head (143a), a piston rod (143b), and an O-ring (143c).

The piston head (143a) may be exposed to an outside of the inlet chamber part (141) and may be formed to receive the external force.

The piston rod (143b) extends from the piston head (143a), and an end portion thereof defines the liquid medicine filling space (141a), and as illustrated in FIG. 2, may include at least one O-ring groove (143b1).

The O-ring (143c) may be configured to be mounted in the O-ring groove (143b1). The O-ring (143c) may include a first O-ring (143c1) and a second O-ring (143c2). In addition, the first and second O-rings (143c1, 143c2) may be formed of the same or different materials. At this time, the O-ring groove (143b1) may include a first O-ring groove (143b1a) and a second O-ring groove (143b1b) respectively corresponding to the first and second O-rings (143c1, 143c2).

Meanwhile, conventionally, it has been recognized that there is no hygienic problem only when a separate configuration such as an elastic membrane is provided so that the liquid medicine filling space (141a) and the piston (143) do not directly contact each other. In contrast, in the present disclosure, the O-ring (143c) may be configured to perform a role of the elastic membrane.

In addition, the piston (143) may be formed of, for example, a plastic material harmless to the human body.

The elastic support member (144) may be formed to elastically support the piston (143).

In addition, the elastic support member (144) may be formed to surround the piston rod (143b) and may be supported by the piston head (143a) and the piston mounting groove (141b1) facing each other, respectively. Accordingly, the elastic support member (144) may be configured to maintain a state in which it is stably disposed at a preset position inside the inlet chamber part (141) without separation.

In addition, the elastic support member (144) may be interposed between the piston head (143a) and the piston mounting groove (141b1) and may provide an elastic force to the piston (143) so as to restore the piston (143), which has moved due to collision with the pressing member (131).

In addition, the elastic support member (144) may be formed to have a ring shape having a hollow portion (144a) in which the piston rod (143b) is accommodated.

The outlet chamber part (145) is coupled to the inlet chamber part (141) and may be configured to discharge the liquid medicine to the outside by pressurization of the piston (143). For example, the outlet chamber part (145) may include an outlet chamber body (145a) and a liquid medicine discharge valve (145b). Here, the liquid medicine discharge valve (145b) may be formed to be in contact with the liquid medicine filled in the liquid medicine filling space (141a).

The outlet chamber part (145) is coupled to the inlet chamber part (141) and may be configured to discharge the liquid medicine to the outside by pressurization of the piston (143).

The outlet chamber body (145a) is coupled to the inlet chamber body (141b) of the inlet chamber part (141) and may include a liquid medicine discharge valve mounting groove (145a1) and a liquid medicine discharge port (145a2) communicating with the liquid medicine discharge valve mounting groove (145a1).

The liquid medicine discharge valve (145b) may be mounted in the liquid medicine discharge valve mounting groove (145a1).

Here, the liquid medicine discharge valve (145b) may be formed to be in contact with the liquid medicine filled in the liquid medicine filling space (141a). In addition, the liquid medicine discharge valve (145b) may be formed to define another portion of the liquid medicine filling space (141a).

The liquid medicine discharge valve (145b) may be formed to selectively open and close the liquid medicine discharge port (145a2) of the outlet chamber body (145a) by a pressure difference, as illustrated in FIG. 4.

In addition, the liquid medicine discharge valve (145b) may be disposed to face the end portion of the piston (143) with the liquid medicine filled in the liquid medicine filling space (141a) interposed therebetween.

In addition, a fluid such as a liquid medicine needs to stably move in a single direction from an inlet to an outlet. Further, a check valve is very commonly used to block or control the flow of the fluid with respect to a direction in which the fluid flows. Such check valves are commercially available in various types and may be easily provided, and it is possible to selectively apply them in consideration of a flow direction, pressure, material, and the like of the fluid.

Here, the end portion of the piston (143) facing the liquid medicine filling space (141a) is configured to define a portion of the liquid medicine filling space (141a). That is, a separate configuration for transmitting the external force of the piston (143) to the liquid medicine filling space (141a) is not provided between the end portion of the piston (143) and the liquid medicine filling space (141a).

According to the configuration of the head (140) described above, the piston (143) that transmits the external force for discharge of the liquid medicine to the liquid medicine filled in the liquid medicine filling space (141a) is configured to directly pressurize the liquid medicine filled in the liquid medicine filling space (141a). That is, the head for a handpiece (140) does not include a separate configuration for transmitting the external force of the piston (143) to the liquid medicine filling space (141a) between the end portion of the piston (143) and the liquid medicine filling space (141a). Accordingly, the external force applied to the piston (143) for discharge of the liquid medicine may be transmitted directly to the liquid medicine filling space (141a). For example, assuming that the magnitude of the external force applied to the piston (143) is 100%, the piston (143) of the present disclosure may stably transmit 85% or more of energy to the liquid medicine filling space (141a).

As a result, by forming the magnitude of the external force applied to the liquid medicine filling space (141a) by the piston (143) to be constant, as compared to a conventional head for a handpiece (140) further including a separate configuration between the piston (143) and the liquid medicine filling space (141a), it is possible to provide a head for a handpiece (140) and a liquid jet injection device (100) including the same having high reliability in terms of accuracy of a liquid medicine administration amount. In addition, it is possible to provide a head for a handpiece (140) and a liquid jet injection device (100) including the same having high energy transmission efficiency by reducing loss of energy transmitted to the liquid medicine by the piston (143).

Hereinafter, the handpiece body (100a) that transmits the external force to the head (140) will be described in more detail.

The handpiece body (100a) is coupled to the head (140) and forms a portion that a user holds by hand when using the liquid jet injection device (100). In addition, the handpiece body (100a) is configured to transmit an external force for discharge of the liquid medicine to the head (140). The handpiece body (100a) includes a cylinder unit (110), a control valve unit (120), and a pressing unit (130).

The cylinder unit (110) includes a cylinder (111), a casing (112), and a hammer (113).

The cylinder (111) is formed to extend in one direction. An inner space of the cylinder (111) is provided such that the hammer (113) is movable between one end and the other end of the cylinder (111). The cylinder (111) may be formed in a cylindrical shape, and both ends thereof may be formed to be open, or only the other end may be formed to be open. In addition, the shape of the cylinder (111) is not limited to a cylindrical shape and may be formed in another polygonal column shape having an inner space for movement of the hammer (113) therein.

The casing (112) is disposed outside the cylinder (111) and is formed to surround the cylinder (111).

The hammer (113) is accommodated inside (111a) of the cylinder (111) and is formed to be movable between one end and the other end of the cylinder (111). The hammer (113) may be formed to correspond to an inner surface of the cylinder (111). For example, when the cylinder (111) has a cylindrical shape, the hammer (113) may also be formed in a cylindrical or columnar shape. Surfaces of the cylinder (111) and the hammer (113) facing each other may be formed to correspond to each other, so that the hammer (113) may stably move while being guided inside the cylinder (111).

The control valve unit (120) may be configured to selectively open and close an intake passage (161) and an exhaust passage (162).

The intake passage (161) forms a passage through which compressed air flows into the cylinder (111). The intake passage (161) is configured to connect a compressed air generation unit (11) generating compressed air and one end of the cylinder (111). That is, one side of the intake passage (161) communicates with the compressed air generation unit (11), and the other side communicates with one end of the cylinder (111). The compressed air generation unit (11) may include a pressure valve (11a) configured to adjust pressure of the compressed air supplied into the inside (111a) of the cylinder (111).

The exhaust passage (162) forms a passage connecting one end of the cylinder (111) and an outside of the cylinder (111). The outside of the cylinder (111) may be an atmospheric region.

A connection passage (163) may be provided between the intake passage (161) and the exhaust passage (162). The connection passage (163) communicates with the inside (111a) of the cylinder (111) and the intake passage (161), and communicates with the inside (111a) of the cylinder (111) and the exhaust passage (162).

The control valve unit (120) may control the connection passage (163) to selectively communicate with either the intake passage (161) or the exhaust passage (162). That is, when the control valve unit (120) communicates the connection passage (163) with the intake passage (161), communication between the connection passage (163) and the exhaust passage (162) may be closed. Conversely, when the control valve unit (120) communicates the connection passage (163) with the exhaust passage (162), communication between the connection passage (163) and the intake passage (161) may be closed.

The control valve unit (120) may perform opening and closing operations of the intake passage (161) and the exhaust passage (162) depending on whether power is applied. For example, when power is applied, the control valve unit (120) may connect the intake passage (161) and the connection passage (163), and when power is cut off, may connect the exhaust passage (162) and the connection passage (163).

The pressing unit (130) includes a pressing member (131) facing an opening formed at the other end of the cylinder (111). The pressing unit (130) may include a pressing unit cover (132) surrounding at least a portion of the pressing member (131).

The head (140) is coupled to the pressing unit (130) and is configured such that, when the hammer (113) collides with the pressing member (131), the liquid medicine stored therein is discharged to the outside by the piston (143) that is pressurized.

The cylinder unit (110), the pressing unit (130), and the head (140) may be formed to be separable from each other and may be provided as a one-time treatment configuration for diversification of injection techniques and prevention of infection.

Meanwhile, a charging part (150) is provided between the casing (112) and the cylinder (111).

The charging part (150) communicates with the other end of the cylinder (111) and forms a space in which compressed air is compressed and charged when the intake passage (161) is opened.

In addition, the charging part (150) may be formed to surround a portion of a side surface of the cylinder (111). For example, based on the liquid jet injection device (100) illustrated in FIG. 2, the charging part (150) may be formed only on a lower portion of the cylinder (111) excluding an upper portion thereof. Accordingly, a shape and/or arrangement of the charging part (150) may be more variously applied, so that a design of the liquid jet injection device (100) may be implemented in more various forms. However, the charging part (150) may be formed to surround an entire side surface of the cylinder (111) rather than only a portion thereof.

Here, when the exhaust passage (162) is opened, the hammer (113) may be configured to return from the other end of the cylinder (111) to one end thereof by the compressed air that is configured to be compressed within the casing (112) when the intake passage (161) is opened.

More specifically, the hammer (113) is configured such that, after moving from one end to the other end of the cylinder (111) by opening of the intake passage (161), when the exhaust passage (162) is opened, the hammer (113) moves from the other end to the one end of the cylinder (111) by the compressed air compressed and charged in the charging part (150).

For example, when the intake passage (161) is opened, the hammer (113) is moved to the other end of the cylinder (111) by the compressed air supplied to one end of the cylinder (111) and collides with the pressing member (131).

Then, after the hammer (113) moves to the other end of the cylinder (111), when the exhaust passage (162) is opened, the compressed air compressed and charged in the charging part (150) may push the hammer (113) toward one end of the cylinder (111) due to an air flow that escapes to the outside of the cylinder (111) through the exhaust passage (162) by a pressure difference between an inside (111a) of the cylinder (111) and the outside thereof.

Meanwhile, the liquid jet injection device (100) may include a controller (12) configured to perform control-related functions such as the control valve unit (120) and the compressed air generation unit (11). The controller (12) may include a display module (not shown) for a user interface.

Meanwhile, a partition plate (123) may be provided between the other end of the cylinder (111) and the pressing member (131), as illustrated in FIG. 6.

A communication part (123a) may be provided in the partition plate (123).

The communication part (123a) is formed to communicate the opening formed at the other end of the cylinder (111) with the charging part (150).

In addition, the communication part (123a) of the partition plate (123) may include a first hole (123a1) communicating with the opening formed at the other end of the cylinder (111), and a second hole (123a2) communicating with the first hole (123a1) and the charging part (150).

According to the structure of the first and second holes (123a1, 123a2), even without providing a separate hole structure to communicate the opening formed at the other end of the cylinder (111) with the charging part (150), a passage through which the compressed air flows between the opening formed at the other end of the cylinder (111) and the charging part (150) may be formed through the first and second holes (123a1, 123a2) by a thickness of the communication part (123a).

Meanwhile, the pressing member (131) is formed to face the opening formed at the other end of the cylinder (111) and to collide with the hammer (113). A pressing member seal (131a) for sealing of the pressing member (131) may be formed on an outer circumferential surface of the pressing member (131).

Meanwhile, a magnetic body (112a) having magnetic force may be provided in the casing (112) facing one end of the cylinder (111). Here, the hammer (113) may be formed to be coupled to the magnetic body (112a) by magnetic force. Accordingly, during an operation in which the hammer (113) moves from the other end of the cylinder (111) back to one end thereof, after the hammer (113) moves back to an initial position, the hammer (113) may stably maintain a state in which it is placed at the initial position.

In addition, at least one of two surfaces of the magnetic body (112a) and the hammer (113) facing each other may include a hammer cushion (113a) formed to absorb an impact when the magnetic body (112a) and the hammer (113) collide with each other.

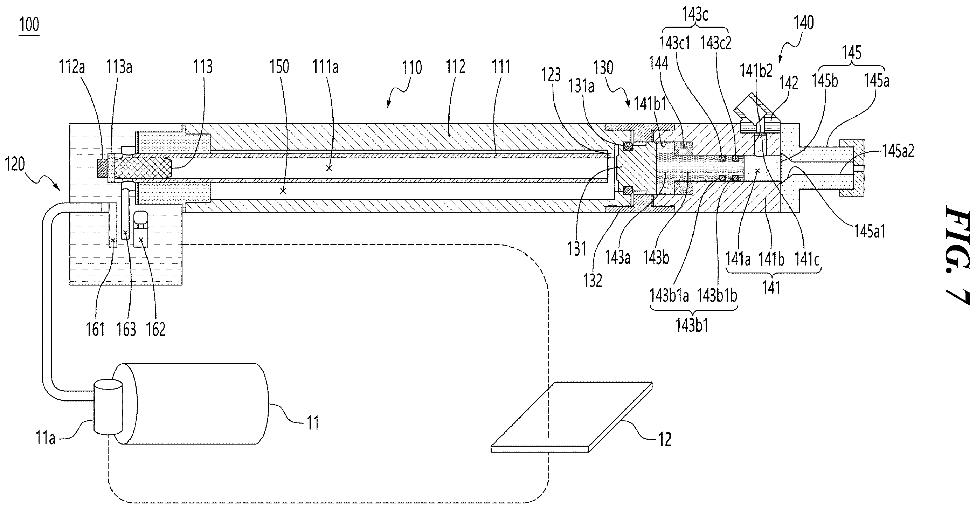

Hereinafter, an operation process of the liquid jet injection device (100) will be described with reference to FIGS. 7 to 11.

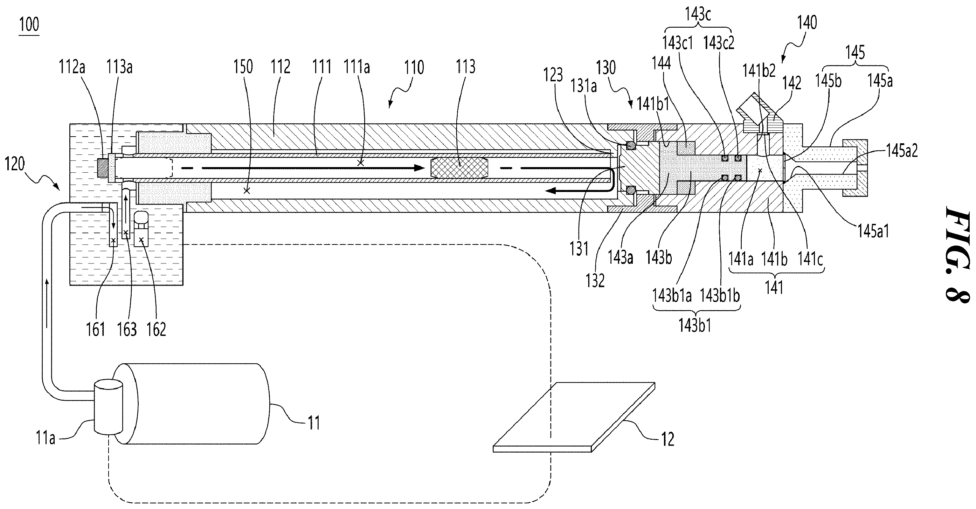

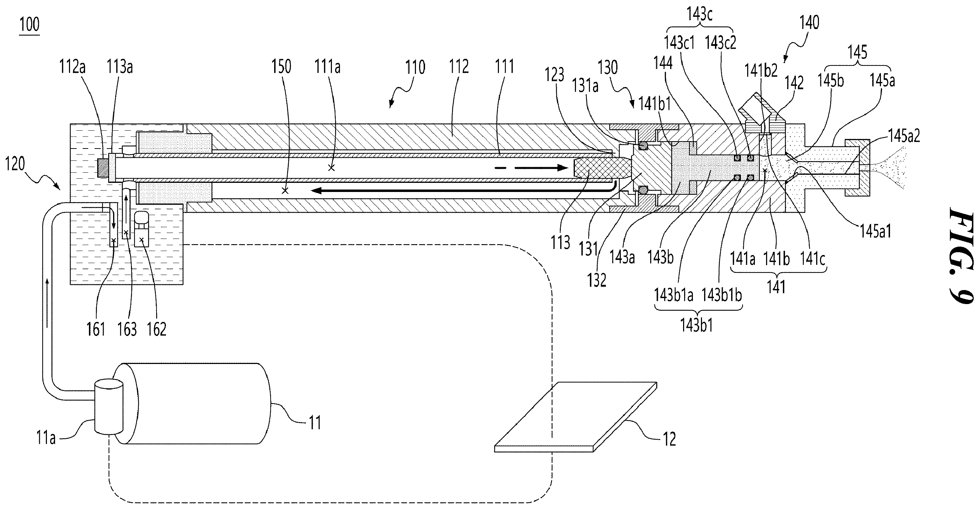

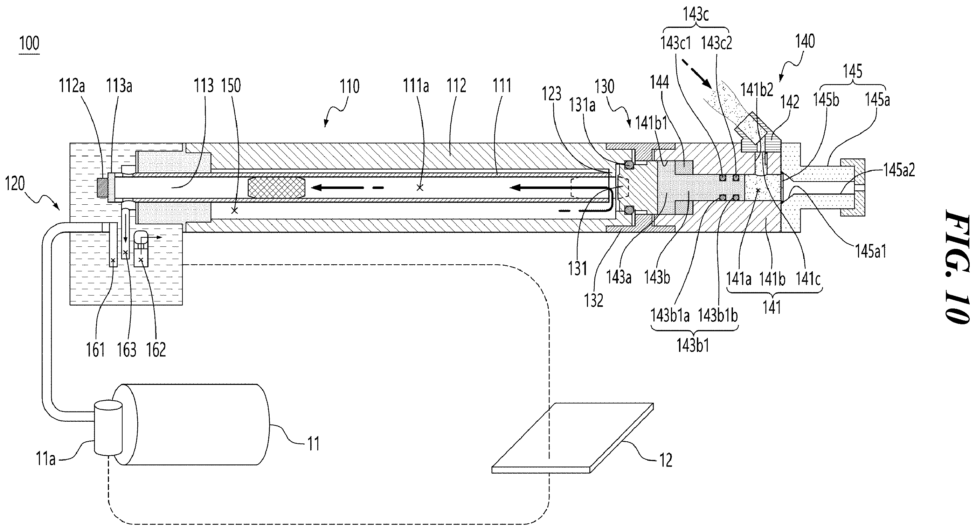

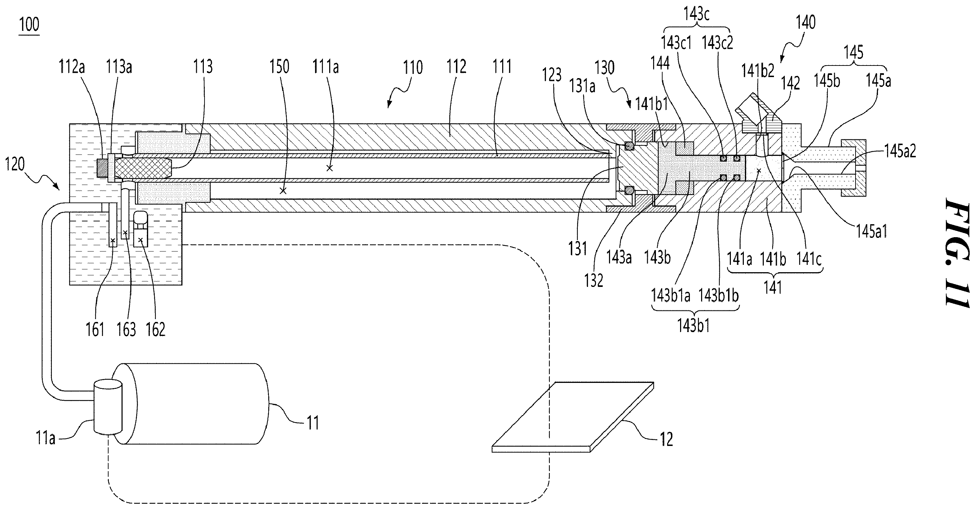

FIG. 7 is a conceptual view illustrating a state before the intake passage (161) of the liquid jet injection device (100) illustrated in FIG. 2 is opened. FIG. 8 is a conceptual view illustrating a state in which the intake passage (161) is opened and the hammer (113) moves from one end of the cylinder (111) toward the other end in the liquid jet injection device (100) illustrated in FIG. 7. FIG. 9 is a conceptual view illustrating a state in which the hammer (113) moves to the other end of the cylinder (111) and collides with the pressing member (131), and the pressing member (131) moves a predetermined distance due to an impact in the liquid jet injection device (100) illustrated in FIG. 8. FIG. 10 is a conceptual view illustrating a state in which, after the hammer (113) collides with the pressing member (131), the exhaust passage (162) is opened and the hammer (113) moves toward one end of the cylinder (111) in the liquid jet injection device (100) illustrated in FIG. 9. FIG. 11 is a conceptual view illustrating a state in which the hammer (113) moves to one end of the cylinder (111) and returns to an initial position in the liquid jet injection device (100) illustrated in FIG. 10.

Referring to FIGS. 7 to 11, compressed air generated by the compressed air generation unit (11) is supplied into the inside (111a) of the cylinder (111), and forward movement of the hammer (113) is generated by air pressure due to the compressed air supplied into the inside (111a) of the cylinder (111). In this manner, air pressure energy is first converted into kinetic energy, the kinetic energy is second converted into collision energy by collision between the hammer (113) and the pressing member (131), and the collision energy due to collision between the pressing member (131) and the piston (143) is third converted again into kinetic energy, so that the liquid medicine filled inside the head (140) is pressurized and discharged through the outlet chamber part (145). Thereafter, as the exhaust passage (162) is opened, the hammer (113) returns to the initial position by a force of the compressed air compressed and charged in the charging part (150) that attempts to escape through the exhaust passage (162).

More specifically, first, referring to FIG. 7, in an initial state of the liquid jet injection device (100), when the intake passage (161) is opened through the control valve unit (120) as illustrated in FIG. 8, compressed air generated by the compressed air generation unit (11) is supplied into the inside (111a) of the cylinder (111) through the intake passage (161). Then, the compressed air supplied into the inside (111a) of the cylinder (111) moves the hammer (113) from one end to the other end of the cylinder (111). At this time, the compressed air is compressed and charged in the charging part (150) formed between the cylinder (111) and the casing (112).

Next, referring to FIG. 9, the hammer (113) moves to the other end of the cylinder (111) by the compressed air and collides with the pressing member (131), and collision energy of the hammer (113) and the pressing member (131) is transmitted to the piston (143) to push the piston (143). At this time, the piston (143), which receives the collision energy, is converted into high-speed fine movement by the elastic support member (144), and pressurizes and pushes the liquid medicine inside the head (140) toward the outlet chamber part (145). Thereafter, as the liquid medicine discharge valve (145b) is pressurized and opened, the liquid medicine inside the head (140) is discharged to the outside through the outlet chamber part (145).

Next, referring to FIG. 10, after pressurization of the piston (143) is completed, negative pressure is generated inside the head (140), and as the liquid medicine injection valve (141c) provided between the feeder part (142) connecting an external liquid medicine storage device (not shown) and the inside of the head (140) is opened, the liquid medicine is filled by a pressure difference between the inside and outside of the head (140). After filling of the liquid medicine is completed and the negative pressure inside the head (140) is removed, the liquid medicine injection valve (141c) automatically performs a closing operation. In addition, as the liquid medicine discharge valve (145b) is closed, it is possible to prevent a phenomenon in which the liquid medicine discharged through the outlet chamber part (145) of the head (140) and blood generated by a wound flow back into the inside.

In addition, as illustrated in FIG. 10, the operation in which the hammer (113) returns to the initial position is achieved through a process in which the compressed air compressed and charged in the charging part (150) formed between the cylinder (111) and the casing (112) pushes the hammer (113) by a force attempting to escape through the exhaust passage (162). In addition, the operation in which the hammer (113) returns to the initial position may be partially achieved by a collision rebound force generated after the hammer (113) collides with the pressing member (131).

Finally, as illustrated in FIG. 11, the hammer (113) returns to the initial position and may stably maintain a state in which it has moved to the initial position by the magnetic body (112a) disposed to face one end of the cylinder (111).

The above description is merely illustrative, and various modifications may be made by those having ordinary skill in the art to which the present disclosure pertains without departing from the scope and technical spirit of the described embodiments. The above-described embodiments may be implemented individually or in any combination.

## Claims

1. A head for a handpiece, the head comprising:
an inlet chamber part coupled to a handpiece body and having a liquid medicine filling space therein;
a feeder part connected to the inlet chamber part and configured to introduce a liquid medicine into the liquid medicine filling space;
a piston mounted inside the inlet chamber part and configured to push a liquid medicine filled in the liquid medicine filling space when pressurized by an external force;
an elastic support member configured to elastically support the piston; and
an outlet chamber part coupled to the inlet chamber part and configured to discharge the liquid medicine by pressurization of the piston,
wherein an end portion of the piston defines a portion of the liquid medicine filling space.

2. The head for a handpiece of claim 1,
wherein the end portion of the piston is in contact with the liquid medicine filled in the liquid medicine filling space.

3. The head for a handpiece of claim 2,
wherein the outlet chamber part comprises:
an outlet chamber body coupled to an inlet chamber body of the inlet chamber part and having a liquid medicine discharge valve mounting groove and a liquid medicine discharge port communicating with the liquid medicine discharge valve mounting groove; and
a liquid medicine discharge valve mounted in the liquid medicine discharge valve mounting groove,
wherein the liquid medicine discharge valve is in contact with the liquid medicine filled in the liquid medicine filling space.

4. The head for a handpiece of claim 3,
wherein the liquid medicine discharge valve is disposed to face the end portion of the piston with the liquid medicine filled in the liquid medicine filling space interposed therebetween.

5. The head for a handpiece of claim 2,
wherein the inlet chamber part comprises:
an inlet chamber body detachably formed on the handpiece body and having a piston mounting groove and a liquid medicine injection valve mounting groove respectively communicating with the liquid medicine filling space at different positions; and
a liquid medicine injection valve mounted in the liquid medicine injection valve mounting groove,
wherein the feeder part is disposed corresponding to the liquid medicine injection valve.

6. The head for a handpiece of claim 5,
wherein the piston comprises:
a piston head exposed to an outside of the inlet chamber part and configured to receive the external force;
a piston rod extending from the piston head, having an end portion defining the liquid medicine filling space, and having at least one O-ring groove; and
an O-ring mounted in the O-ring groove.

7. The head for a handpiece of claim 6,
wherein the elastic support member is formed to surround the piston rod and is respectively supported by the piston head and the piston mounting groove facing each other.

8. The head for a handpiece of claim 7,
wherein the elastic support member has a ring shape having a hollow portion in which the piston rod is accommodated.

9. A liquid jet injection device comprising:
a handpiece body; and
a head for a handpiece according to any one of claims 1 to 8, coupled to the handpiece body,
wherein the handpiece body comprises:
a cylinder unit including a cylinder, a casing surrounding the cylinder, and a hammer accommodated inside the cylinder and configured to move between one end and the other end of the cylinder;
a control valve unit configured to selectively open and close an intake passage connecting a compressed air generation unit generating compressed air supplied into the cylinder and one end of the cylinder, and an exhaust passage connecting one end of the cylinder and an outside; and
a pressing unit including a pressing member facing an opening formed at the other end of the cylinder and coupled to the inlet chamber part,
wherein, when the hammer collides with the pressing member, the pressing member is configured to press the piston.

10. The liquid jet injection device of claim 9,
wherein a charging part is provided between the casing and the cylinder, communicates with the other end of the cylinder, and is configured such that compressed air is compressed and charged therein when the intake passage is opened,
wherein the hammer is configured to move from the other end to the one end of the cylinder by the compressed air charged in the charging part when the exhaust passage is opened, after having moved from the one end to the other end of the cylinder upon opening of the intake passage.
